# EUROPEAN PATENT SPECIFICATION

(11) **EP 1 210 346 B1**
(45) Date of publication and mention of the grant of the patent: **02.07.2003**
(21) Application number: 00958872.4
(22) Date of filing: 11.09.2000
(51) Int. Cl.: C07D 471/18, A61K 31/4995, A61P 35/00

(54) **NEW ACTIVE MARINE ALKALOIDS**
AKTIVE MARINE ALKALOIDEN
NOUVEAUX ALCALOIDES MARINS ACTIFS

(30) Priority: 10.09.1999 GB 9921477
(43) Date of publication of application: 05.06.2002
(73) Proprietor: Instituto Biomar S.A., 24231 Onzonilla, Leon (ES)
(72) Inventor: CIMINO, Guido, Inst. Chimica di Molecole, I-80072 Arco Felice (IT); FONTANA, Angelo, Inst.Chimica di Molecole, I-80072 Arco Felice (IT); GARCIA GRAVALOS, Dolores, Pharma Mar, S.A., E-28760 Madrid (ES); WAHIDULLA, Solimabi, Nat. Inst. of Oceanography, Goa 403 004 (IN); NAIK, Chandrakant, Govind, Nat.Inst.Oceanography, Goa 403 004 (IN)
(74) Representative: Ruffles, Graham Keith
(86) International application number: GB0003489
(87) International publication number: WO01019824

(56) References cited:
- US-A- 5 023 184
- HE, HAI YIN ET AL: "Renieramycins E and F from the sponge Reniera sp.: reassignment of the stereochemistry of the renieramycins" J. ORG. CHEM. (1989), 54(24), 5822-4 , XP002162279
- FONTANA, A. ET AL: "A new antitumor isoquinoline alkaloid from the marine nudibranch Jorunna funebris" TETRAHEDRON (2000), 56(37), 7305-7308 , XP002162280

## Description

The present invention relates to new active alkaloids and in particular to an alkaloid isolated from the mollusc *Jorunna funebris*.

Marine organisms, especially soft corals, sponges and tunicates, provide many secondary metabolites and exhibit a varying degree of biological activity (Faulkner, D.J., *Nat. Prod. Reports*, **1999,** *16*, 155-198 and references cited therein).

Faulkner, D.J., and He, H.-Y., *J. Org. Chem*., **1989,** *54*, 5822-5824, discloses the compounds renieramycins E and F and their isolation from the sponge *Reniera sp.*

### Summary of the Invention

The present invention provides alkaloids having the following formula (I): 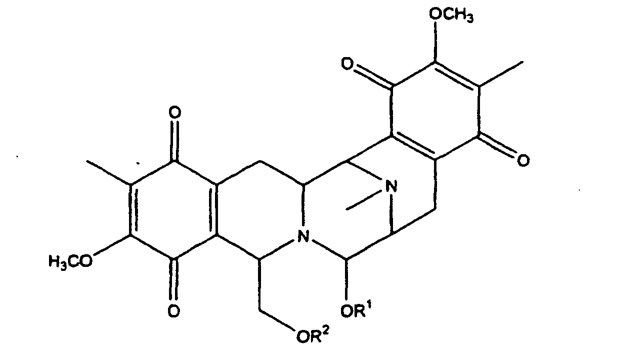 wherein R¹ is selected from the group consisting of hydrogen, alkyl having 1 to 6 carbon atoms and alkanoyl having 2 to 7 carbon atoms, and R² is selected from the group consisting of hydrogen and alkanoyl having 2 to 7 carbon atoms.

In the definitions of the groups in formula (I), the alkyl group and the alkyl moiety of the alkanoyl group are a straight-chain or branched alkyl group having 1 to 6 carbon atoms such as methyl, ethyl, propyl, isopropyl, butyl, isobutyl, sec-butyl, tert-butyl, pentyl, neopentyl and hexyl.

Jorumycin exhibits antitumour activity. In particular, Jorumycin exhibits antitumour activity against cell lines derived from human solid tumours, such as human lung carcinoma, human colon carcinoma and human melanoma, and the like. It is active against other tumour cell lines, like leukaemia and lymphoma.

The present invention further provides pharmaceutical compositions which contain as active ingredient a compound of the formula (I), as well as a process for its preparation.

The present invention also provides use of the compound of formula (I) in the preparation of a medicament for the treatment of prophylaxis of tumours.

More particularly, the present invention relates to Jorumycin (R¹=H and R²=Ac in formula (I)), extracted and isolated from the mollusc *Jorunna funebris*. Jorumycin is of the formula:

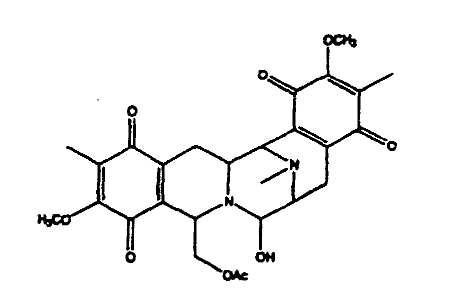

Jorumycin free from other compounds occurring in the mollusc is provided, as well as substantially pure jorumycin. A further aspect of the invention is a method for preparing the compound Jorumycin (R¹=H and R²=Ac in the formula (I)), which comprises extraction and isolation from the mollusc *Jorunna funebris*.

### Preferred Embodiments of the Invention

Examples of pharmaceutical compositions include any solid (tablets, pills, capsules, granules, etc.) or liquid (solutions, suspensions or emulsions) with suitable formulation of oral, topical, parenteral or other administration, and they may contain the pure compound or in combination with any carrier or other pharmacologically active compounds. These compositions may need to be sterile when administered parenterally.

The correct dosage of a pharmaceutical composition comprising compounds of the formula (I), will vary according to the pharmaceutical formulation, the mode of application, and the particular situs, host and tumour being treated. Other factors like age, body weight, sex, diet, time of administration, rate of excretion, condition of the host, drug combinations, reaction sensitivity and severity of the disease shall be taken into account. Administration can be carried out continuously or periodically within the maximum tolerated dose.

### Antitumour Activity

Cells were maintained in logarithmic phase of growth in Eagle's Minimum Essential Medium, with Earle's Balanced Salts, with 2.0 mM L-glutamine, with non-essential amino acids, without sodium bicarbonate (EMEM/neaa); supplemented with 10% Fetal Calf Serum (FCS), 10⁻² M sodium bicarbonate and 0.1 g/l penicillin-G + streptomycin sulfate.

A screening procedure has been carried out to determine and compare the antitumour activity of these compounds, using an adapted form of the method described by Bergeron et al (Raymond J Bergeron, Paul F Cavanaugh, Jr, Steven J Kline, Robert G Hughes, Jr, Gary T Elliot and Carl W Porter. Antineoplastic and antiherpetic activity of spermidine catecholamide iron chelators. *Biochem. Bioph. Res. Comm*. **1984,** *121*, 848-854). The antitumour cells employed were P-388 (suspension culture of a lymphoid neoplasm from DBA/2 mouse), A-549 (monolayer culture of a human lung carcinoma), HT-29 (monolayer culture of a human colon carcinoma) and MEL-28 (monolayer culture of a human melanoma).

P-388 cells were seeded into 16 mm wells at 1 x 10⁴ cells per well in 1 ml aliquots of MEM SFCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, an approximately IC₅₀ was determined by comparing the growth in wells with drug to the growth in wells control.

A-549, HT-29 and MEL-28 cells were seeded into 16 mm wells at 2 x 10⁴ cells per well in 1 ml aliquots of MEM 10FCS containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, the wells were stained with 0.1% Crystal Violet. An approximately IC₅₀ was determined by comparing the growth in wells with drug to the growth in wells control.

The results are given in the following table:

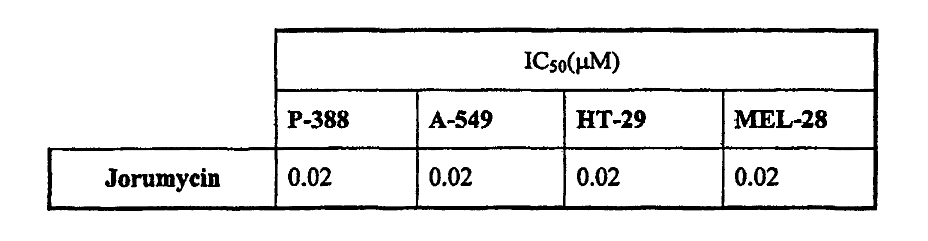

### Antitumour Activity

Jorumycin showed also activity against Gram-positive bacteria (*Staphylococcus* and others).

### Extraction and Isolation

Jorumycin was isolated from the mollusc *Jorunna funebris* (Mollusca: Nudibranchia:

Doridina: Kentrodorididae) collected off Mandapam (India) in April 1998. The product is present in the extract of both the mucus and mantle of the nudibranch. In a typical procedure, the frozen biological sample (mucus or animal body) was extracted with acetone. After removing the organic solvent at reduced pressure the residue was partitioned between water (15 ml) and ethyl acetate. The water phase was extracted for three times with ethyl acetate (total 34 ml). The oily residue obtained by removing of the organic solvent at reduced pressure was fractioned by Sephadex LH-20 chromatography following the elution of the extract components by SiO₂-TLC (CHC₁₃/MeOH 95:5, Rf= 0.4). The final purification of Jorumycin was obtained by sequential SiO2 chromatographies (CHCl₃/MeOH) and HPLC (Sherisorb S5W analytical column, isocratic elution with: n-hexane/CHC₁₃/TEA 90:10:10, detector: Waters R401 differential refractometer). The product is soluble in CHC₁₃, CH₂Cl₂, MeOH. It is highly unstable in acid and basic media.

### Jorumycin:

(C₂₇H₃₀N₂O₉);
[α]_{D}= -57° (*c* 0.05 CHCl₃);
IR (liquid film) 3310, 1731, 1700 cm⁻¹;
UV (MeOH) 268 nm (ε=15000);
¹H-NMR (CDCl₃) δ 1.25 (m, 1H), 1.75 (s, 3H), 1.93 (s, 3H), 1.96 (s, 3H), 2.24 (d, 1H, J=20.1 Hz), 2.26 (s, 3H), 2.65 (dd, 1H, J=20.1 and 7.3 Hz), 2.84 (dd, 1H, J=16.7 and 2.1 Hz) 3.16 (bdt, 1H, J=12.0, 2.7 and 2.7 Hz). 3.18 (bs, 1H), 3.82 (dd, 1H, J=11.2 and 3.5 Hz), 3.90 (bs, 1H), 3.98 (s, 3H), 4.00 (bs, 3H), 4.36 (bm, 1H), 4.40 (bs, 1H), 4.42 (dd, 1H, J=11.2 and 3.7 Hz);
¹³C-NMR (CDCl₃) δ 185.8 (s), 181.3 (s), 170.0 (s), 155.7 (s), 155.0 (s), 141.9 (s), 141.7 (s), 137.2 (s), 128.8 (s), 128.4 (s), 113.8 (s), 109.4 (s), 83.0 (d), 65.2 (t), 61.0 (d), 57.7 (d), 56.1 (d), 54.4 (d), 52.6 (d), 41.3 (q), 25.5 (t), 20.7 (q), 20.6 (t), 8.8 (q), 8.7 (q);ESMS (*m*/*z*) 526 (20, M+), 508 (100, M-H₂O), 494 (10, M-32);
HRESMS (*m*/*z*) 508.189 (Δ + 5 mmu).

Other compounds of formula (I) may readily be prepared by chemical synthesis or hemisynthesis.

## Claims

1. A compound of the following formula (I): 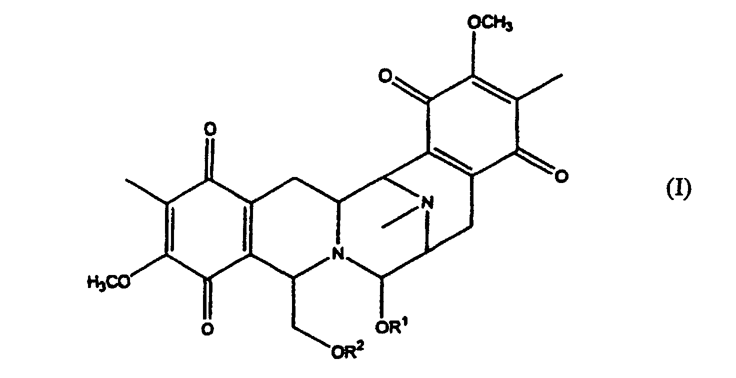 wherein R¹ is selected from the group consisting of hydrogen, alkyl having I to 6 carbon atoms and alkanoyl having 2 to 7 carbon atoms and R² is selected from the group consisting of hydrogen and alkanoyl having 2 to 7 carbon atoms.

2. A compound according to claim 1, which is jorumycin, where R¹ is H and R² is Ac:

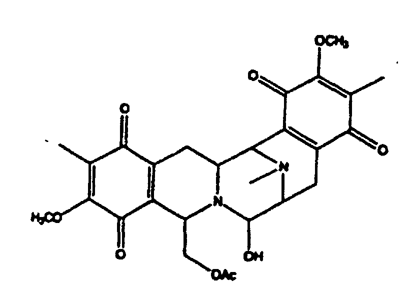

3. A pharmaceutical composition comprising a compound of the formula (I) of claim 1, together with a pharmaceutically acceptable carrier.

4. A method for preparing the compound of claim 2, which comprises extraction and isolation from the mollusc *Jorunna funebris.*

5. The use of a compound of formula (I) of claim 1 in the preparation of a medicament for the treatment of tumours.

## Patentansprüche

1. Verbindung der folgenden Formel (I): 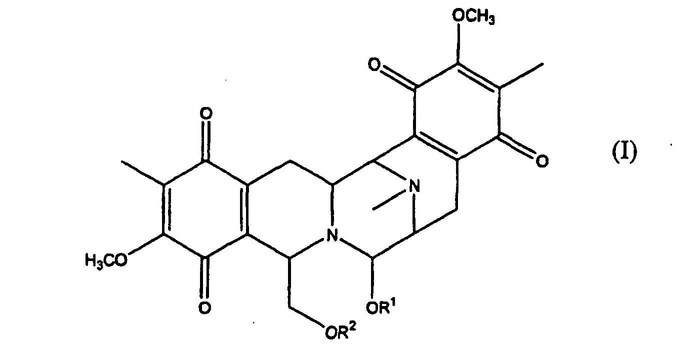 worin R¹ ausgewählt wird aus der Gruppe, bestehend aus Wasserstoff, Alkyl mit 1 bis 6 Kohlenstoffatomen und Alkanoyl mit 2 bis 7 Kohlenstoffatomen und R² ausgewählt wird aus der Gruppe, bestehend aus Wasserstoff und Alkanoyl mit 2 bis 7 Kohlenstoffatomen.

2. Verbindung nach Anspruch 1, die Jorumycin ist, worin R¹ H ist und R² ist Ac:

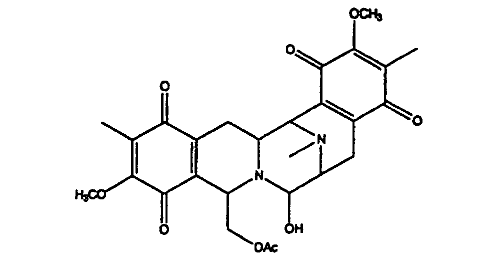

3. Pharmazeutische Zusammensetzung, aufweisend eine Verbindung der Formel (I) nach Anspruch 1 zusammen mit einem pharmazeutisch zulässigen Träger.

4. Verfahren zum Herstellen der Verbindung nach Anspruch 2, welches Verfahren die Extraktion und Isolation aus dem Weichtier *Jorunna funebris* umfasst.

5. Verwendung einer Verbindung der Formel (I) nach Anspruch 1 in der Herstellung eines Medikaments für die Behandlung von Tumoren.

## Revendications

1. Composé de la formule (I) suivante: 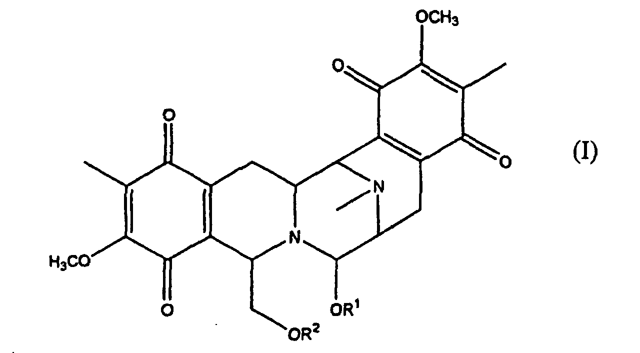 dans laquelle R' est choisi dans le groupe constitué d'un hydrogène, d'un groupe alkyle contenant de 1 à 6 atomes de carbone et alcanoyle contenant de 2 à 7 atomes de carbone et R² est choisi dans le groupe constitué d'un hydrogène et d'un groupe alcanoyle contenant de 2 à 7 atomes de carbone.

2. Composé suivant la revendication 1, qui est la jorumycine, dans laquelle R¹ est H et R² est Ac:

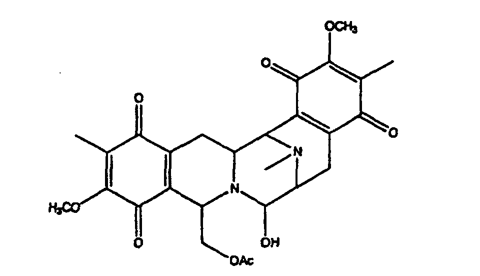

3. Composition pharmaceutique comprenant un composé de la formule (I) suivant la revendication 1, accompagné d'un excipient pharmaceutiquement acceptable.

4. Procédé pour la préparation du composé suivant la revendication 2, qui comprend une extraction et un isolement à partir du mollusque *Jorunna funebris*.

5. Utilisation d'un composé de la formule (I) suivant la revendication 1, dans la préparation d'un médicament pour le traitement de tumeurs.
